# EUROPEAN PATENT SPECIFICATION

(11) **EP 2 648 678 B1**
(45) Date of publication and mention of the grant of the patent: **04.10.2017**
(21) Application number: 11784639.4
(22) Date of filing: 15.11.2011
(51) Int. Cl.: A61K 8/06, A61K 8/27, A61K 8/34, A61K 8/46, A61Q 11/00, A61Q 17/00, A61K 8/19

(54) **AN ANTIMICROBIAL MICROEMULSION MOUTHWASH COMPOSITION**
ANTIMIKROBIELLE MUNDWASSERZUSAMMENSETZUNG IN FORM EINER MIKROEMULSION
COMPOSITION DE BAIN DE BOUCHE ANTIMICROBIEN SOUS FORME DE MICROÉMULSION

(30) Priority: 07.12.2010 IN 3321MU2010; 25.01.2011 EP 11151948
(43) Date of publication of application: 16.10.2013
(73) Proprietor: Unilever N.V., 3013 AL Rotterdam (NL); Unilever PLC, a company registered in England and Wales under company no. 41424, Greater London EC4Y 0DY (GB)
(72) Inventor: ROUT, Deeleep, Kumar, Bangalore 560 066 (IN); SAJI, Maya, Treesa, Bangalore 560 066 (IN); SINHA, Ritesh, Kumar, Bangalore 560 066 (IN)
(74) Representative: van den Brom, Coenraad Richard
(86) International application number: PCT/EP2011/070093
(87) International publication number: WO 2012/076295

(56) References cited:
- EP-A1- 2 047 889
- WO-A1-95/12379
- WO-A2-2008/060130
- DE-A1- 19 509 079
- US-A- 4 992 259
- US-A- 5 283 056
- US-A- 5 474 761
- US-A- 5 817 295

## Description

### Technical Field

The invention relates to a transparent/translucent liquid antimicrobial mouthwash composition that is substantially free of low molecular weight alcohol.

### Background of the invention

Oral hygiene is one of the most important aspect of personal care among consumers. Consumers all over the world use different types of products for oral care. People routinely brush their teeth with a toothbrush and a dentifrice which may be a toothpaste or a toothpowder at least two times a day. Use of such brushing ensures maintaining good oral hygiene by minimising oral bacteria that accumulate in the mouth over the course of sleeping in the night or during the course of the day when people eat their food and consume beverages. Brushing, thus minimises problems like cavities, tartar, gingivitis, caries, and bad breath, also known as halitosis.

In spite of brushing teeth twice a day, many people suffer from various forms of one or more of the above named diseases and this is believed to be caused by bacteria acting in the oral mucosa over the about twelve hour period between brushing. During such times, people resort to rinsing/ gargling their mouth with an antiseptic mouthwash.

Although most antiseptic mouthwashes are aqueous based, many of them contain a substantial amount of alcohol (e.g. ethyl alcohol or isopropyl alcohol) which are the best known anti-microbial actives which ensure fast kinetics of bacterial kill. However, there are many countries where culturally or otherwise, there is reluctance to using alcohol in mouthwashes. Most known antibacterial actives do not provide as fast a kill as alcohol. Further, people prefer mouthwashes which have a transparent or translucent appearance.

The present inventors have in WO20101046238 disclosed a fast acting antimicrobial composition comprising thymol and terpineol. It is a problem to include these or other antimicrobial actives which are generally substantially water insoluble materials in an aqueous composition which is transparent or translucent. One way of including oils in water while ensuring transparency is to formulate them in a microemulsion composition. However microemulsion compositions generally require high amounts of surfactants and solvents especially low molecular weight alcohol. It is thus a challenge to formulate a mouthwash composition that is on the one hand effective in killing microbes while on the other hand has a transparent/ translucent appearance while being substantially free of low molecular weight alcohol.

US 5283056 discloses an alcohol-free transparent mouthwash consisting of an alcohol-free mouthwash base:, water and a stable transparent oil-in-water microemulsion flavor concentrate consisting of:(i) water;(ii) one or more hydrophobic flavor oils; and (iii) one or more surfactants in the absence of lower alkanols wherein the mixing ratio of water, oil and surfactant is defined according to the shaded area of a phase diagram which has been shown as Figure 1-A of US5283056. This patent utilises high amounts of surfactant, generally higher than 10% and in most cases of the order of 20 to 30% surfactant which is mostly non-ionic surfactant to maintain the microemulsion stability.

The present inventors have been working on solving the problem of providing a microemulsion mouthwash composition which includes low amount of surfactant while meeting the objective of being especially effective in killing *S*. *mutans,* a bacteria responsible for diseases in oral cavity. The composition is not only appealing since it has a transparent/ translucent appearance but also includes only ingredients which most people will have no aversion to using to clean their mouths.

### Summary of the Invention

According to one aspect of the present invention there is provided a transparent/translucent liquid antimicrobial mouthwash composition in micro emulsion format substantially free of C1 to C3 alcohol comprising:
(i) 0.05% to 10 % of an antimicrobial active selected from terpineol, thymol, eugenol, borneol, or limonene;
(ii) 0.05 to 10% of a surfactant;
(iii) 0.01 to 50% of a di-or multivalent electrolyte selected from calcium chloride, magnesium chloride, zinc sulphate or zinc acetate; and
(iv) 50 to 99.9% water;
wherein the surfactant is of the anionic or cationic class.

According to preferred aspect of the present invention there is provided a composition of the first aspect for disinfecting oral mucosa.

According to another preferred aspect of the present invention there is provided a composition of the first aspect for use in a method of disinfecting the oral mucosa comprising the steps of rinsing the mouth/ oral mucosa with the composition of the invention.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Except in the operating and comparative examples, or where otherwise explicitly indicated, all numbers in this description indicating amounts of material or conditions of reaction, physical properties of materials and/or use are to be understood as modified by the word "about". Unless specified otherwise, numerical ranges expressed in the format "from x to y" are understood to include x and y. When for a specific feature multiple preferred ranges are described in the format "from x to y", it is understood that all ranges combining the different endpoints are also contemplated.

The invention provides for a transparent/translucent liquid antimicrobial composition in microemulsion format capable of acting as a mouthwash for disinfecting the oral cavity. The composition is unique in that it is substantially free of low boiling alcohols like ethanol or IPA (isopropyl alcohol) while ensuring high microbial kill in short time frames. The composition may be used as such (without dilution) or may be diluted with water before rinsing or washing mouth. The composition comprises an antimicrobial active, a surfactant, an electrolyte and water.

The microemulsion composition of the invention is preferably transparent. This format of the composition is especially preferred since the transparent form makes for an appealing visual impact on consumers. In a preferred aspect, the dispersed phase droplets in the microemulsion are in the size range of 6 to 125 nm, more preferably in the size range of 6 to 15 nm.

The antimicrobial active is selected from the class comprising terpenes, essential oils, cationic oils, C6 to C10 fatty acids, fatty acid methyl esters, or organic per-acids having a solubility in water of less than 2000 ppm at 25 °C. Of the above classes, the more preferred ones are terpenes, essential oils, C6 to C10 fatty acids or organic per-acids, a further more preferred list includes terpenes, essential oils, or organic per-acids and most preferred actives are the class of terpenes or essential oils.

The following are the preferred list of actives from each class.

### Terpenes/ Essential oils:

Amyl salicylate, carvacrol, cymene, e.g. p-cymene, dihydroeugenol, eugenol, hexyl eugenol, hexyl salicylate, isoeugenol, methyl eugenol, methyl isoeugenol, methyl salicylate, tert butyl cresol, thymol, and vanillin. Examples of non-aromatic essential oils of terpenoid compounds include cedrene, cineole, citral (including geranial and neral), citronellal, citronellol, eucalyptol (also known as 1,8 cineole) paradihydrolinalool, dihydromyrcenol (DH myrcenol), farnesol, geraniol, hexyl cinnamaldehyde, hydroxycitronallol, hydroxycitronellal, isocitral, limonene, preferably d-limonene, linalool, longifolene, menthol, nerol, nerolidiol, pinene, e.g. α-pinene, phellendrene, terpinene, e.g. α-terpinene and γ-terpinene, terpineol, e.g. γ-terpineol and terpin-4-ol, and tetrahydromyrcenol (THM).

### Cationic oils:

Quaternary Ammonium Cationic Vegetble Oil and Charged aminopolydimethylsilane of the chemical formula (CH₃)₃-Si-[Si (CH₃)₂-O]-[Si(CH₃)-((CH₂)₃-NH-(CH₂)₂-NH₂)-O]₂-Si-(CH₃)₃.

### Fatty acids:

have the general structure R-COOH Where R is a hydrocarbon chain which may be branched or linear with carbon atom varying from 6-10.

### Fatty acid methyl esters:

have the general structure R-COOR' Where R and R' are hydrocarbon chains which may be branched or linear with carbon atoms such that the sum of the number of carbon atoms in R and R' is greater than or equal to 7.

### Organic per-acids:

Examples of organic acids suitable for use in the composition of the invention are ε-phthalimidoperoxyhexanoic acid, diperoxyketals, peroxydicarbonates peroxyesters, diacyl preoxides, benzoyl peroxide, ketone peroxides, dialkyl peroxides, hydroperoxides.

More preferred antimicrobial actives are terpineol, thymol, eugenol, borneol, limonene, iso-borneol, eucalyptol, camphor or mixtures thereof. Most preferred antimicrobial actives are terpineol, thymol, eugenol, borneol or limonene. An especially preferred composition comprises thymol and terpineol. An even further preferred composition comprises thymol, terpineol and eugenol. The antimicrobial active is present in 0.05 to 10%, more preferably 0.05 to 5%, more preferably 0.1 to 5% by weight of the microemulsion composition.

The microemulsion composition of the invention comprises 0.05 to 10%, preferably 0.05 to 8 %, more preferably 0.5 to 5% surfactant by weight of the composition. The surfactant is of the cationic or anionic class, more preferably of the cationic class. When anionic surfactant is present it is preferably chosen from alkali or alkaline earth metal salts of alkyl sulphonic acid, fatty acid, or alkyl ether sulphate. When a cationic surfactant is present it is benzalkonium chloride, alkyl pyridinium chloride or quaternary ammonium gemini surfactants.

Without wishing to be bound by theory it is believed that the advantages of the invention accrue from the synergistic interaction between the anionic/ cationic surfactant at the low concentrations used with the anti-microbial agent to provide the desired anti-microbial efficacy in the oral cavity while the low concentration of the surfactant interacting with the electrolye provides the desired microemulsion stability.

The microemulsion composition of the invention is substantially free of low molecular weight alcohol viz. C1 to C3 alcohols e.g. ethanol or isopropyl alcohol. By the term substantially free of low molecular weight alcohols is meant that the alcohol is present in an amount which does not significantly affect the microbial kill. Preferably C1 to C3 alcohols is present in less than 2%, more preferably less than 1% and most preferably absent from the composition of the invention.

The composition of the invention preferably comprises 50 to 99.9%, preferably 50 to 80%, further more preferably 50 to 70 % water by weight of the composition.

The composition of the invention includes an electrolyte at 0.01 to 50% by weight of the composition. Preferred electrolyte may be chosen from a chloride, sulphate, acetate, or carbonante of an alkali, alkaline earth or transition metal. Of these, multivalent electrolyes are especially preferred. More useful electrolytes include chloride, sulphate or acetate of an alkaline earth metal. Examples of electrolytes useful in including in the composition of the invention are calcium chloride, sodium chloride, magnesium chloride, zinc sulphate, aluminium chloride or zinc acetate. The more preferred electrolytes are calcium chloride, magnesium chloride, zinc sulphate or zinc acetate while the most preferred electrolytes are zinc sulphate or zinc acetate. Electrolyte is preferably present in 0.01 to 30%, more preferably 0.01 to 10%, further more preferably 0.05 to 5% by weight of the composition. Without wishing to be bound by theory it is believed that another advantage for inclusion of the electrolyte in the microemulsion composition of the invention in addition to meeting all of the objects of the invention is to ensure that low molecular weight alcohols are substantially absent from the composition.

The microemulsion composition of the invention is to be used for disinfecting the oral mucosa either by using the composition as-is i.e with no dilution or after diluting the composition with water. The preferred weight ratio of composition to water for the dilution step is in the range of 1:1 to 1:200, more preferably 1:5 to 1:50 and optimally 1:15 to 1:30 and ideally about 1:20. By the term transparent or translucent composition is meant that the composition has a turbidity value of 0 to 200 NTU.

The composition of the invention preferably has a pH ranging from 3 to 8, more preferably from 3.5 to 8 at 25 °C.

According to another preferred aspect of the invention there is provided a composition as per the present invention for use in a method of disinfecting the oral mucosa comprising the step of rinsing the mouth/ oral mucosa with the composition. The composition of the invention is preferably diluted with water in a ratio in the range of 1:1 to 1:200 before the rinsing step. The predetermined time for rinsing the mouth/ oral mucosa with the composition may be up to two minutes, preferably up to one minute and more preferably up to 30 seconds.

According to another preferred aspect of the invention there is provided a composition according to the present invention for disinfecting oral mucosa. The use is preferably non-therapeutic.

The invention will now be illustrated with the help of the following non-limiting examples.

### EXAMPLES

### Examples 1-5: Efficacy of various mouthwash compositions against 5. mutans in a one minute contact test in suspension

*Streptococcus mutans* (*S. mutans)* is a Gram-positive, facultatively anaerobic bacterium commonly found in the human oral cavity. *S. mutans* is the leading cause of dental caries (tooth decay) worldwide and is considered to be the most cariogenic of all of the oral streptococci. *S.mutans,* sticks to the surface of teeth and subsists on a diverse group of carbohydrates. While metabolising sugar and other energy sources, the microbe produces acid that causes cavities in teeth

Various compositions as shown in Table - 1 were prepared.

The compositions listed in Table - 1 were used to test the efficacy as a antibacterial mouth wash composition against S. *mutans* in a one minute contact test in suspension, using the following procedure.

Sub culture *S.mutans* was taken in a BHI broth at 37 grams per liter (gpl) and it was allowed to grow in CO₂ incubator (15% CO₂) at 37°C for 15 hours. The S. *mutans* culture was adjusted to a optical density to 0.3 (∼10⁸ cfu/ml) at 620nm. 9ml of the desired composition was taken and added to 1ml of culture and mixed. After a one minute contact time with the culture, they were neutralized in D/E broth and after serial dilution they were plated in BHI Agar (52gpl). The plates were incubated in CO₂ incubator. The residual colonies were counted after 48 hours incubation. The efficacy was compared against a control sample.

The amount of log reduction of the bacteria was measured and the data is summarized in Table - 1.

**Table - 1**

| **Ingredient,** | **Example 1^{@} Wt%** | **Example 2 Wt%** | **Example 3 Wt%** | **Example 4 Wt%** | **Example 5 Wt%** |
|---|---|---|---|---|---|
| Terpineol | - | 0.125 | 0.125 | - | - |
| Thymol | 0.06 | 0.250 | 0.250 | - | - |
| Eugenol | - | 0.025 | 0.025 | - | - |
| SLES# | - | 0.400 | - | 0.400 | - |
| Zinc Sulphate | - | 0.120 | - | - | 0.120 |
| Eucalyptol | 0.09 | - | - | - | - |
| Menthol | 0.04 | - | - | - | - |
| Ethanol | 21.6 | - | - | - | - |
| Water | To 100 | To 100 | To 100 | To 100 | To 100 |
| Log reduction | 7.6 | 7.6 | 3.8 | 0.2 | 0.2 |

| | | | | | |
|---|---|---|---|---|---|
| @ Example - 1 is a composition used by a well known mouthwash sold under the brand name Listerine # SLES is the anionic surfactant Sodium Lauryl ether sulphate. | | | | | |

The data in Table - 1 indicates that the antibacterial efficacy of the composition of the invention (Example - 2) is comparable with a commercially available composition which has high levels of alcohol (Example - 1). Further the data also indicates that neither the mixture of antibacterial actives in water (Example - 3), nor the surfactant (Example - 4) nor the electrolyte (Example - 5) in themselves are not efficacious in providing the antibacterial efficacy.

### Examples 6 to 12: Efficacy of various mouthwash compositions against S. mutans in a thirty second contact test in suspension

Various compositions as shown in Table - 2 were prepared.

The compositions listed in Table - 2 were used to test the efficacy as a antibacterial mouth wash composition against S. *mutans* in a thirty second contact test in suspension, using the same procedure as used for Examples 1 to 5 except that the time of contact was 30 seconds instead of one minute. The data is summarized in Table - 2.

**Table - 2**

| Ingredient | Ex - 6 Wt% | Ex - 7 Wt% | Ex - 8 Wt% | Ex - 9 Wt% | Ex - 10 Wt% | Ex - 11& Wt% |
|---|---|---|---|---|---|---|
| Terpineol | 0.125 | - | - | 0.125 | 0.603 | - |
| Thymol | 0.250 | - | - | 0.250 | 0.125 | 0.06 |
| Eugenol | 0.025 | - | - | 0.025 | 0.013 | - |
| SLES | - | 0.400 | - | 0.400 | 0.200 | - |
| Zinc Sulphate | - | - | 0.120 | 0.120 | 0.06 | - |
| Eucalyptol | - | - | - | - | - | 0.09 |
| Menthol | - | - | - | - | - | 0.04 |
| Ethanol | - | - | - | - | - | 21.6 |
| Water | To 100 | To 100 | To 100 | To 100 | To 100 | To 100 |
| Log reduction | 1.0 | 0.2 | 0.3 | 7.1 | 7.1 | 7.1 |

| | | | | | | |
|---|---|---|---|---|---|---|
| ^{&} Example - 11 is the Listerine composition. | | | | | | |

The data in Table -2 indicates that the results of Table - 1 are valid even for a thirty second test.

### Examples 12 to 17: Efficacy of various mouthwash compositions against S. mutans biofilm

Various compositions as shown in Table - 3 were prepared.

The compositions listed in Table - 3 were used to test the efficacy against *S. mutans* biofilm, using the following procedure.

Sub culture *S.mutans* in BHI broth (37gpl) was allowed to grow in CO₂ incubator (15% CO₂) at 37 °C for 15 hours. The culture was processed and the optical density was adjusted to 0.3 (∼10⁸ cfu/ml) in BHI broth at 620nm. Glucose (2%) was added to the processed culture and it was dispensed on a 6 well plate and allowed to grow in a CO₂ incubator. The media was replaced every 24 hours to make a 3 days old biofilm. The media was removed and about 2ml of the various compositions of Table - 3 were added to the biofilm. After one minute contact time, the compositions were removed and 2ml of neutralizer was added immediately. The biofilm cells were removed with a cell scraper. The biofilm cells were mixed and serial diluted in D/E neutralizer and plated at 1ml of the sample in BHI Agar. The plates were incubated in CO₂ incubator for 48hrs and the colonies were counted.

The efficacy of the mouthwash compositions in terms of log reduction are summarized in Table - 3.

**Table - 3**

| **Ingredient** | **Ex- 12 Wt%** | **Ex- 13 Wt%** | **Ex- 14 Wt%** | **Ex- 15 Wt%** | **Ex- 16 Wt%** | **Ex- 17^{&} Wt%** |
|---|---|---|---|---|---|---|
| Terpineol | 0.125 | - | - | 0.125 | 0.603 | - |
| Thymol | 0.250 | - | - | 0.250 | 0.125 | 0.06 |
| Eugenol | 0.025 | - | - | 0.025 | 0.013 | - |
| SLES# | - | 0.400 | - | 0.400 | 0.200 | - |
| Zinc Sulphate | - | - | 0.120 | 0.120 | 0.06 | - |
| Eucalyptol | - | - | - | - | - | 0.09 |
| Menthol | - | - | - | - | - | 0.04 |
| Ethanol | - | - | - | - | - | 21.6 |
| Water | To 100 | To 100 | To 100 | To 100 | To 100 | To 100 |
| Log reduction | 0.9 | 0.9 | 0.1 | 5.6 | 3.8 | 5.6 |

The data in Table - 3 indicates that the results of Table - 1 and Table - 2 are valid even when tested on a biofilm.

### Examples 18 to 23: Various other preferred mouthwash compositions of the invention

Various mouthwash compositions of the invention were prepared as shown in Table - 4 and found to have the desired efficacy.

**Table - 4**

| **Examples** | **18** | **19** | **20** | **21** | **22** | **23** |
|---|---|---|---|---|---|---|
| Terpineol | 0.25 | 1 | 0.25 | 1 | 1 | |
| Thymol | 0.125 | 0.5 | 0.125 | 0.5 | 0.5 | - |
| Eugenol | 0.025 | 0.1 | 0.025 | 0.1 | 0.1 | - |
| Methyl caprylate | - | - | - | - | - | 6 |
| SLES | 0.4 | - | 0.4 | - | - | - |
| Benzalkonium chloride (dilution >=1:20 in use) | 0 | 3 | - | 3 | 3 | 3 |
| Zinc sulphate | 0.06 | - | - | - | - | 0.6 |
| Sodium chloride | - | 0.2 | 0.13 | 0 | 0 | - |
| Magnesium chloride | - | - | - | - | 0.85 | - |
| Calcium chloride | - | - | - | 0.6 | - | - |
| water | To 100 | To 100 | To 100 | To 100 | To 100 | To 100 |

Examples 19 and 20 are not according to the invention. The present invention thereby provides for a mouthwash composition which is highly effective and yet substantially free of alcohol. The composition is also highly appealing since it has a transparent appearance.

## Claims

1. A transparent/translucent liquid antimicrobial mouthwash composition in micro emulsion format substantially free of C1 to C3 alcohol comprising:
(i) 0.05% to 10 % of an antimicrobial active selected from terpineol, thymol, eugenol, borneol, or limonene;
(ii) 0.05 to 10% of a surfactant;
(iii) 0.01 to 50% of a di- or multivalent electrolyte selected from calcium chloride, magnesium chloride, zinc sulphate or zinc acetate; and
(iv) 50 to 99.9% water
wherein the surfactant is of the anionic or cationic class.

2. A composition as claimed in claim 1 wherein said surfactant is an alkali or alkaline earth metal salt of alkyl sulphonic acid or fatty acid; or alkyl ether sulphate.

3. A composition as claimed in any one of the preceding claims comprising 0.01 to 10 % electrolyte.

4. A composition as claimed in any one of the preceding claims for use in disinfecting oral mucosa.

5. A composition as claimed in any one of the preceding claims for use in a method of disinfecting the oral mucosa comprising the steps of rinsing the mouth or oral mucosa with the composition.

6. A composition as claimed in claim 5 for use in a method of disinfecting oral mucosa wherein in said method the composition is diluted with water in a ratio in the range of 1:1 to 1:200 before the rinsing step.

## Patentansprüche

1. Transparente/durchscheinende flüssige antimikrobielle Mundwasserzusammensetzung im Format einer Mikroemulsion, die im Wesentlichen frei von C1- bis C3-Alkohol ist, umfassend:
(i) 0,05% bis 10% eines antimikrobiellen Wirkstoffs, ausgewählt aus Terpineol, Thymol, Eugenol, Borneol oder Limonen,
(ii) 0,05 bis 10% eines Tensids,
(iii) 0,01 bis 50% eines zwei- oder mehrwertigen Elektrolyten, ausgewählt aus Calciumchlorid, Magnesiumchlorid, Zinksulfat oder Zinkacetat, und
(iv) 50 bis 99,9% Wasser,
worin das Tensid aus der anionischen oder kationischen Klasse stammt.

2. Zusammensetzung, wie im Anspruch 1 beansprucht, wobei das Tensid ein Alkali- oder Erdalkalimetallsalz von Alkylsulfonsäure oder Fettsäure oder ein Alkylethersulfat darstellt.

3. Zusammensetzung, wie in irgendeinem der vorhergehenden Ansprüche beansprucht, umfassend 0,01 bis 10% Elektrolyt.

4. Zusammensetzung, wie in irgendeinem der vorhergehenden Ansprüche beansprucht, zur Verwendung beim Desinfizieren von Mundschleimhaut.

5. Zusammensetzung, wie in irgendeinem der vorhergehenden Ansprüche beansprucht, zur Verwendung in einem Verfahren zum Desinfizieren von Mundschleimhaut, umfassend die Schritte des Spülens des Mundes oder der Mundschleimhaut mit der Zusammensetzung.

6. Zusammensetzung, wie im Anspruch 5 beansprucht, zur Verwendung in einem Verfahren zum Desinfizieren von Mundschleimhaut, wobei in dem Verfahren die Zusammensetzung vor dem Spülschritt mit Wasser in einem Verhältnis in dem Bereich von 1:1 bis 1:200 verdünnt wird.

## Revendications

1. Composition d'eau de bouche antimicrobienne liquide transparente/translucide dans un format de micro-émulsion pratiquement exempte d'alcool en C1 à C3 comprenant :
(i) de 0,05 % à 10 % d'une matière active antimicrobienne choisie parmi le terpinéol, le thymol, l'eugénol, le bornéol, ou le limonène ;
(ii) de 0,05 à 10 % d'un tensioactif ;
(iii) de 0,01 à 50 % d'un électrolyte di- ou multivalent choisi parmi le chlorure de calcium, le chlorure de magnésium, le sulfate de zinc ou l'acétate de zinc ; et
(iv) de 50 à 99,9 % d'eau
où le tensioactif est de la classe anionique ou cationique.

2. Composition selon la revendication 1, où ledit tensioactif est un sel de métal alcalin ou alcalino-terreux d'acide alkylesulfonique ou d'acide gras ; ou un sulfate d'alkyléther.

3. Composition selon l'une quelconque des revendications précédentes comprenant de 0,01 à 10 % d'électrolyte.

4. Composition selon l'une quelconque des revendications précédentes pour une utilisation dans la désinfection de muqueuses orales.

5. Composition selon l'une quelconque des revendications précédentes destinée à une utilisation dans un procédé de désinfection des muqueuses orales comprenant les étapes de rinçage de la bouche ou des muqueuses orales avec la composition.

6. Composition selon la revendication 5 destinée à une utilisation dans un procédé de désinfection de muqueuses orales où dans ledit procédé la composition est diluée avec de l'eau dans un rapport dans l'intervalle de 1:1 à 1:200 avant l'étape de rinçage.
